(19) Europäisches Patentamt
European Patent Office
Office européen des brevets

(11) Veröffentlichungsnummer: **0 002 719**
**B1**

(12) **EUROPÄISCHE PATENTSCHRIFT**

(45) Veröffentlichungstag der Patentschrift: **24.06.81**

(51) Int. Cl.³: **C 07 D 237/14**

(21) Anmeldenummer: **78101651.4**

(22) Anmeldetag: **13.12.78**

(54) **Verfahren zur Herstellung von 3-Pyridazonen.**

(30) Priorität: **24.12.77 DE 2757923**

(43) Veröffentlichungstag der Anmeldung: **11.07.79 Patentblatt 79/14**

(45) Bekanntmachung des Hinweises auf die Patenterteilung: **24.06.81 Patentblatt 81/25**

(84) Benannte Vertragsstaaten: **BE CH DE FR GB NL**

(56) Entgegenhaltungen:
**FR-A-1 570 521**
**GB-A-1 184 862**
**REAGENTS FOR ORGANIC SYNTHESIS BY L.F. Fieser, M. Fieser (J. Wiley, New York), 1967,**

(73) Patentinhaber: **HOECHST AKTIENGESELLSCHAFT, Zentrale Patentabteilung Postfach 80 03 20, D-6230 Frankfurt/Main 80 (DE)**

(72) Erfinder: **Müller, Werner Heinrich, Dr., Taunusblick 5, D-6239 Eppstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG.

# Verfahren zur Herstellung von 3-Pyridazonen

3-Pyridazone werden als Zwischenprodukte für die Synthese von Pharmaka und Pflanzenschutzmitteln eingesetzt (z.B. Brit. Patent 1 157 045, DE-OS 2 331 398). Es ist bekannt, substituierte 6-Phenyl-3-pyridazone durch Umsetzung von Benzoylacrylsäuren mit Hydrazin herzustellen (J. Chem. Soc. 1965, 3347). Die Ausbeuten sind hierbei jedoch niedrig, da bei der üblichen Synthese von Benzoylacrylsäuren hauptsächlich die trans-Isomeren gebildet werden, die im Unterschied zu den cis-Isomeren nicht in der gewünschten Weise reagieren. Bei der Umsetzung der unsubstituierten Benzoylacrylsäuren mit Hydrazin bildet sich nach Gabriel et al., Ber. 32, 396 kein 6-Phenyl-3-pyridazon, sondern das Hydrazon der Benzoylacrylsäure.

Es ist ferner bekannt, 6-Phenyl-3-pyridazone aus ihren 4,5-Dihydroderivaten durch Oxidation mit elementarem Brom zu gewinnen (J. Am. Chem. Soc. 75 (1953) 11117, Ber. 32, 399). Der Nachteil dieses Verfahrens ist, dass die Pyridazone als Hydrobromide anfallen und aus diesen erst durch Behandlung mit Basen freigesetzt werden müssen. Die dabei entstehenden Salze müssen aus Kostengründen und um eine Umweltbelastung zu vermeiden, durch aufwendige Aufarbeitung des Abwassers zurückgewonnen werden.

Weiterhin ist 6-Methyl-3-pyridazon bereits durch Dehydrierung von 6-Methyl-4,5-dihydro-3-pyridazon mit salpetriger Säure oder mit Chromtrioxid in 10 bzw. 20%iger Ausbeute erhalten worden (W.G. Overend and L.F. Wiggins, J. Chem. Soc. 1947, 239).

Ausserdem ist noch bekannt, Aminophenylpyridazone durch Oxidation ihrer 4,5-Dihydroderivate mit Nitrobenzolsulfonsäure zu gewinnen (DE-OS 1 670 043).

Alle die erwähnten Oxidationsmittel sind teuer, schlecht zu handhaben und bedeuten, wenn sie ins Abwasser gelangen, eine erhebliche Umweltbelastung.

Es wurde nun ein Verfahren zur Herstellung von 3-Pyridazonen der allgemeinen Formel

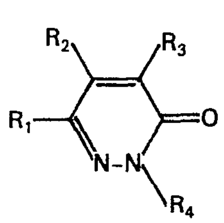

gefunden, wobei die Reste $R_1$ bis $R_4$ gleich oder verschieden sein können und Wasserstoff, geradkettige, verzweigte oder cyclische Alkylgruppen oder Arylgruppen bedeuten können, das dadurch gekennzeichnet ist, dass man 4,5-Dihydro-3-pyridazone der allgemeinen Formel

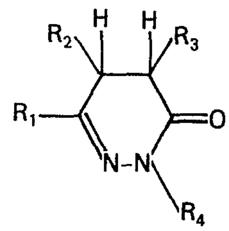

worin $R_1$ bis $R_4$ die oben genannte Bedeutung haben, in der Flüssigphase in Gegenwart eines Dehydrierungskatalysators, der Palladium, Platin, Ruthenium, Rhodium oder Iridium enthält, auf eine Temperatur von 150 bis 350 °C erhitzt.

Als geradkettige, verzweigte oder cyclische Alkylgruppen sind im allgemeinen solche mit bis zu 12 C-Atomen geeignet, insbesondere solche mit bis zu 6 C-Atomen.

Weiterhin können die Alkylreste auch noch substituiert sein, beispielsweise durch Halogene, insbesondere Fluor, Chlor oder Brom oder durch die Phenyl-, Naphthyl-, Hydroxy-, Alkoxy- oder Trifluormethyl-Gruppe. Geeignete Arylgruppen sind beispielsweise Phenyl- oder durch Halogen-, Hydroxy-, Alkoxy- oder Alkylgruppen mit bis zu 6 C-Atomen oder durch Trifluormethyl- oder Pentafluoräthylgruppen substituierte Phenylreste.

Die Edelmetalle Palladium, Platin, Ruthenium, Rhodium und Iridium werden im allgemeinen auf Trägern verwendet, wie z.B. Kohle, Aluminiumoxide, Kieselsäure, Alumosilikat, Magnesiumsilikat, Chromoxid, Chromia-Alumina, Spinellen, Zeolithen, Magnesiumoxid, Calciumoxid, Titandioxid und Asbest. Vorzugsweise verwendet man Palladium und/oder Platin auf Kohle, $SiO_2$, $Al_2O_3$, Chromia-Alumina, Alumosilikaten, Spinellen oder Zeolithen. Die Konzentration des Edelmetalles beträgt dann zweckmässigerweise 0,02 bis 20 Gew.-%, bezogen auf das Gewicht des Trägers, vorzugsweise 0,1 bis 10 Gew.-%. Vorzugsweise enthält der Katalysator neben dem Edelmetall noch Chrom als Cokatalysator, und zwar besonders bevorzugt 0,1 bis 20 Gew.-% bezogen auf das Gewicht des Trägers.

Es war unerwartet, dass Pyridazone gemäss dem erfindungsgemässen Verfahren mit für eine Dehydrierung ungewöhnlich hohen Ausbeuten selbst bei sehr oft wieder eingesetztem Katalysator gewonnen werden können, da bekannt ist, dass Stickstoffverbindungen häufig Katalyysatorgifte für Edelmetallkatalysatoren darstellen.

Die für das erfindungsgemässe Verfahren notwendigen Ausgangsmaterialien erhält man in ausgezeichneten Ausbeuten durch Umsetzung der entsprechenden Ketosäuren, -ester oder -nitrile mit einem Hydrazinhydrat gemäss folgendem Schema:

(R = H oder Alkyl)

1. 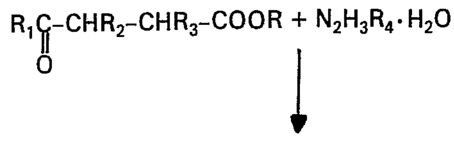

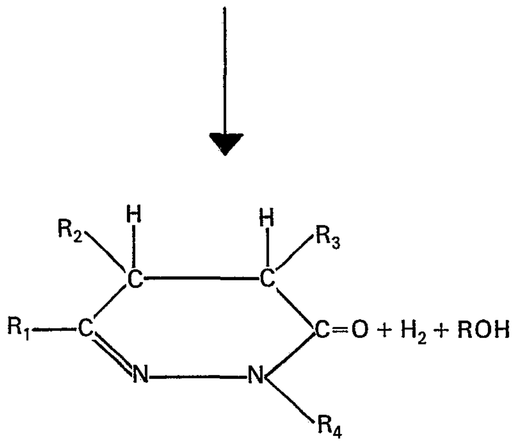

2. $R_1C\text{-}CHR_2\text{-}CHR_3\text{-}CN + N_2H_3R_4 \cdot H_2O$

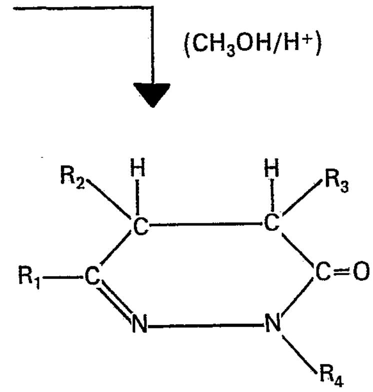


Für die Herstellung der hierbei eingesetzten Ketosäuren, Ketoester bzw. Ketonitrile bieten sich die Friedel-Crafts Acylierung von Aromaten mit Bernsteinsäureanhydrid bzw. die Addition von Acrylnitrilen oder Acrylestern an Aldehyde unter Cyanid- oder Thiazoliumkatalyse an:

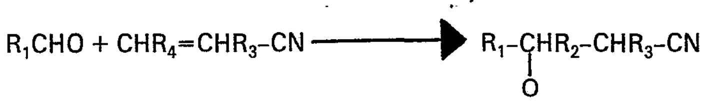

$R_1CHO + CHR_2{=}CHR_3\text{-}COOR \longrightarrow R_1C\text{-}CHR_2\text{-}CHR_3\text{-}COOR$

Das erfindungsgemässe Verfahren kann diskontinuierlich oder kontinuierlich durchgeführt werden, und zwar bei Temperaturen zwischen 150 und 350 °C. Vorzugsweise werden jedoch Temperaturen von 180 bis 270 °C angewandt, da bei diesen Temperaturen eine besonders hohe Selektivität bei sehr raschem Ablauf der Dehydrierung erreicht wird.

Der Reaktionsdruck beträgt im allgemeinen 0,01 bis 20 bar.

Vorzugsweise wird der Partialdruck des sich bei der Dehydrierung bildenden Wasserstoffs niedrig gehalten, damit das Gleichgewicht zugunsten der Dehydrierung verschoben und eine Hydrierung der Ausgangsverbindungen und Endprodukte vermieden wird. Dieser niedrige Wasserstoffpartialdruck kann erzielt werden durch Spülen des Reaktionssystems mit einem Inertgas, wie Stickstoff, Argon oder Kohlendioxid. Vorzugsweise arbeitet man in Gegenwart eines Lösungsmittels. Als Lösungsmittel eignen sich beispielsweise Kohlenwasserstoffe, aliphatische und aromatische Äther, Ester und Amide. Bevorzugte Lösungsmittel sind aliphatische und aromatische Äther, Ester und Amide, z.B. Äthylenglykol-, Diäthylenglykol-, Triäthylenglykol-dialkyläther, Diphenyläther, Äthylenglykoldiacetat, Propandioldiacetat, Butandioldiacetat, Diäthylenglykoldiacetat, Cyclohexylpropionat, Bernsteinsäuredialkyl-($C_1$–$C_6$)ester, Glutarsäure-($C_1$–$C_6$)dialkylester, Dimethylformamid, Dimethylacetamid, N-Methylpyrrolidon, N-Methyl-Formanilid, Adipinsäure-($C_1$–$C_6$)dialkylester.

Besonders bevorzugt sind Diäthylenglykol- und Triäthylenglykoldialkyläther, die Alkylgruppen mit bis zu 6 C-Atomen haben, z.B. Diäthylenglykoldimethyläther (Diglyme), Triäthylenglykoldimethyläther (Triglyme), Diäthylenglykoldiäthyläther, Triäthylenglykoldiäthyläther, Diäthylenglykoldibutyläther, Diäthylenglykolmethyl-butyl-äther (= Methylbutyldiglykol), Triäthylenglykolmethyl-butyl-äther, sowie Diphenyläther.

Das erfindungsgemässe Verfahren kann beispielsweise mit einem Festbett-Trägerkatalysator oder mit einem Trägerkatalysator durchgeführt werden, der durch Rühren in der Reaktionslösung suspendiert gehalten wird.

Bei diskontinuierlicher Reaktionsführung wird im allgemeinen das Reaktionsgemisch aus Lösungsmittel, Katalysator und Dihydropyridazon solange unter Rühren erhitzt – vorzugsweise bei Rückfluss – bis sich kein Abgas mehr entwickelt. Sodann wird der Rührer abgestellt, die Wasserstoffatmosphäre durch Stickstoff verdrängt und die noch heisse Lösung vom abgesetzten Katalysator getrennt. Beim Abkühlen kristallisiert das reine Pyridazon aus und wird durch Filtration gewonnen. Die Mutterlauge wird durch frisches Dihydropyridazon ergänzt und in den Reaktor zum Katalysator zurückgegeben. Nach 10 bis 15 Versuchen wird frisches Lösungsmittel verwendet, das durch Destillation der Mutterlauge gewonnen wird. Die Nebenprodukte befinden sich im Destillationsrückstand.

Bei kontinuierlicher Reaktionsführung wird im allgemeinen eine Lösung eines Dihydropyridazons kontinuierlich in den Dehydrierreaktor, der einen fest angeordneten oder durch Rühren suspendierten Katalysator enthält, eingebracht, während gleichzeitig eine entsprechende Menge der Reaktionsmischung, welche im wesentlichen das gebildete Pyridazon enthält, ausgetragen wird. Der Katalysator wird dabei entweder mittels einer Abscheidevorrichtung im Reaktor zurückgehalten oder nach Abtrennung von der Reaktionsmischung, z.B. mittels einer Zentrifuge

oder eines Dekanters, in den Reaktor zurückgeführt. Beim Abkühlen der ausgetragenen Reaktionsmischung kristallisiert das gebildete Pyridazon aus und wird durch Filtration gewonnen. Bei Verwendung der oben als besonders bevorzugt genannten Lösungsmittel (Di- oder Triäthylenglykoldialkyläther) zeigt sich überraschenderweise, dass die Dihydropyridazone besser löslich sind als die herzustellenden Pyridazone und deswegen letztere aus der Reaktionslösung rein ausfallen, selbst wenn der Umsatz nicht vollständig ist. Da der Hauptteil der Mutterlauge nach Zugabe von frischem Dihydropyridazon sowieso wieder zurückgeführt wird, ist es also nicht notwendig, die Reaktion bis zum vollständigen Umsatz zu betreiben, was eine erhebliche Zeitersparnis bedeutet. Als besonders vorteilhaft hat sich natürlich erwiesen, dass das Pyridazon aus den bevorzugten Lösungsmitteln besonders leicht auskristallisiert und so die energieintensive destillative Entfernung des Lösungsmittels entfällt. Bei kontinuierlicher Reaktionsführung wird nur ein geringer Anteil der Mutterlauge nach Auskristallisation des Pyridazons ausgeschleust und destillativ aufgearbeitet. Das dabei rein gewonnene Lösungsmittel wird in den Prozess zurückgegeben.

Das vorliegende Verfahren gestattet es also, die leicht zugänglichen Dihydropyridazone in einer technisch einfach durchzuführenden katalytischen Reaktion glatt in Pyridazone zu überführen. Die Pyridazone werden in guter Ausbeute gebildet und können auf einfache Weise aus der Reaktionslösung isoliert werden. Die für die Reaktion verwendeten Katalysatoren haben gute Aktivität, Stabilität und Standzeit.

Beispiele

Herstellung der Dihydropyridazone

a) 6-Phenyl-4,5-dihydro-3-pyridazon[1)]

178 g (1 Mol) β-Benzoylpropinsäure[2)] wurden in einer Lösung von 62,5 g 80%igem Hydrazinhydrat (1 Mol) in 500 ml Wasser gelöst und zwei Stunden auf 100°C erhitzt. Nach dem Abkühlen wurden die weissen Kristalle abgenutscht und mit kaltem Wasser gewaschen. Ausbeute 170 g = 97,2% d.Th., Fp. 151°C.

b) 6-Methyl-4,5-dihydropyridazon wurde entsprechend aus Lävulinsäure und Hydrazinhydrat hergestellt. Dabei fällt das Produkt zuerst als Hydrat (Fp. 80°C) an, das im Vakuumtrockenschrank bei 80 bis 100°C entwässert wird (Fp. 103°C).

[1)] hergestellt nach Ingolf Crossland et al., Acta Chemica Scandinavica 19, (1965) 1652-1660

[2)] hergestellt mit 90% Ausbeute nach Org. Syn. Coll. Vol. II (1943) 81

Herstellung der Pyridazone

Beispiel 1

In einem 250 ml Dreihalskolben, ausgerüstet mit Rührer, Thermometer, Rückflusskühler und Gasauffanggefäss, wurden 100 ml Methylbutyldiglykol zusammen mit 10 g 6-Phenyl-4,5-dihydro-3-pyridazon und 10 g eines Katalysators, der 1,3 Gew.-% Palladium auf Chromia-Aluminia (19 Gew.-% $Cr_2O_3$ auf hochaktiver Tonerde $Al_2O_3$) enthielt, 5 Stunden am Rückfluss (215 bis 220°) erhitzt, wobei sich 1,5 l Gas entwickelten. Nach Abstellen des Rühres liess man auf 150 bis 160°C abkühlen, wobei sich der Katalysator absetzte; anschliessend wurde die über dem Katalysator stehende Lösung abgetrennt und auf 50°C gekühlt, worauf sich 3,8 g 6-Phenyl-3-pyridazon abschieden (Fp. 190°C).

Der Mutterlauge wurden weitere 10 g Ausgangssubstanz zugesetzt, dann wurde sie wieder in den Dreihalskolben zum Katalysator gegeben. Nach Wiederholung des Versuchs schieden sich 8,3 g 6-Phenylpyridazon mit Fp. 198°C ab. Nach der 2. Wiederholung betrug die Ausbeute 94% und der Fp. 200°C.

Nach der 20. Wiederholung betrug die Ausbeute noch 90% und der Fp. 195°C. Bei der 21. Wiederholung wurde frisches Methylbutyldiglykol verwendet und die Mutterlauge ausgeschleust. Die Ausbeute betrug diesmal nur 62%, der Fp. war jedoch wieder auf 198°C gestiegen. Zwischen der 22. und 30. Wiederholung betrug die Ausbeute 95%, der Fp. war bei der 30. Wiederholung auf 190°C gefallen. Nach Austausch der Mutterlauge gegen frisches Lösungsmittel stieg der Fp. wieder auf 198°C. Nach der 40. Wiederholung betrug die Ausbeute 93% bei einem Fp. von 194°C. Zu diesem Zeitpunkt war keine Abnahme der Aktivität oder Selektivität des Katalysators zu erkennen, vielmehr war die Aktivität im Lauf der Versuche gestiegen, so dass die für die Dehydrierung benötigte Zeit von anfangs 5 Stunden auf 2 Stunden gefallen war. Besonders überraschend dabei ist, dass der Katalysator nicht vergiftet wird, obwohl man den Anteil der Nebenprodukte in der Mutterlauge erheblich ansteigen lässt, indem man die Mutterlauge nur etwa nach jedem 10. Versuch durch neues Lösungsmittel ersetzt.

Beispiel 2

Versuchsausführung wie Beispiel 1, jedoch mit 2,6 g Katalysator, der 5 Gew.-% Pd auf $Al_2O_3$ enthielt.

Nach jedem 10. Versuch wurde die Mutterlauge durch frisches Methylbutyldiglykol ersetzt.

Die durchschnittliche Ausbeute bis zum 25. Versuch betrug 92%. Die Schmelzpunkte nahmen von 200°C (frisches Methylbutyldiglykol) bis auf 195°C (kurz vor dem Ausschleusen der Mutterlauge) ab. Die Reaktionszeit nahm von anfangs 4 Stunden auf 3 Stunden ab.

Beispiel 3

Versuchsausführung wie in Beispiel 1, jedoch mit 13 g Katalysator, der 1 Gew.-% Pd auf $SiO_2$ (120 $m^2$ BET-Oberfläche) enthielt.

Die durchschnitliche Ausbeute, für 20 Versuche ermittelt, betrug 96,2%. Der Schmelzpunkt war von 198°C auf 190°C beim 13. Versuch gefallen und stieg nach Lösungsmittelerneuerung bei Versuch 14 wieder auf 198°C an. Die Reaktionszeit betrug 3 bis 4 Stunden. Das Abgas bestand zu 99% aus Wasserstoff.

Beispiel 4

Versuchsausführung wie Beispiel 1, jedoch mit

26 g Katalysator, der 0,5 Gew.-% Pd und 1,0 Gew.-% Cr auf Lithium-Aluminiumspinell enthielt. Die durchschnittliche Ausbeute vom 3. bis 8. Versuch betrug 97,8% 6-Phenylpyridazon mit einem Schmelzpunkt von 198 °C.

Beispiel 5

Versuchsausführung wie Beispiel 1, jedoch mit 100 ml Diäthylenglykoldiäthyläther (Siedepunkt 192 °C) als Lösungsmittel. Die Ausbeute stieg von 42% im 1. auf 92% im 3. Versuch, Fp. 198 °C.

Beispiel 6

Versuchsausführung wie Beispiel 1, jedoch mit 100 ml Diphenyläther als Lösungsmittel, wobei 2,5 Stunden bei 255 °C am Rückfluss erhitzt wurde.

Die Ausbeute stieg von 68% im 1. auf 89% im 3. Versuch, Fp. 197 °C.

Beispiel 7

Versuchsausführung wie Beispiel 1, jedoch mit 10 g 6-Methyl-4,5-dihydro-3-pyridazon (Fp. 103 °C). Es wurde 5 Stunden am Rückfluss (215 bis 218 °C) erhitzt, wobei sich etwa 2,5 l Gas entwikkelten.

Danach wurde die Reaktionslösung bei 130 °C vom Katalysator abgetrennt, auf 50 °C abgekühlt und vom auskristallisierten 6-Methyl-3-pyridazon abfiltriert. Das Filtrat wurde nach Zugabe von 10 g 6-Methyl-4,5-dihydro-3-pyridazon in den Reaktionskolben zurückgegeben.

Die Ausbeute an 6-Methyl-3-pyridazon betrug nach dem 1. Versuch 66%, nach dem zweiten 68% und nach dem dritten 92% der Theorie, Fp. 136 bis 139 °C.

**Patentansprüche**

1. Verfahren zur Herstellung von 3-Pyridazonen der allgemeinen Formel

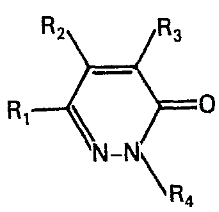

wobei die Reste $R_1$ bis $R_4$ gleich oder verschieden sein können und Wasserstoff, geradkettige, verzweigte oder cyclische Alkylgruppen oder Arylgruppen bedeuten können, dadurch gekennzeichnet, dass man 4,5-Dihydro-3-pyridazone der allgemeinen Formel

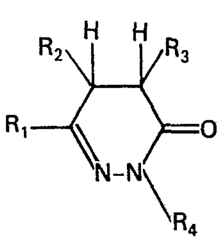

worin $R_1$ bis $R_4$ die oben genannte Bedeutung haben, in der Flüssigphase in Gegenwart eines Dehydrierkatalysators, der Palladium, Platin, Ruthenium, Rhodium oder Iridium enthält, auf eine Temperatur von 150 bis 350 °C erhitzt.

2. Verfahren nach Anspruch 1, dadurch gekennzeichnet, dass man die Reaktion bei einem Druck von 0,01 bis 20 bar ausführt.

3. Verfahren nach Anspruch 1, dadurch gekennzeichnet, dass das Edelmetall auf einem Träger aufgebracht ist.

4. Verfahren nach Anspruch 3, dadurch gekennzeichnet, dass die Konzentration des Edelmetalls 0,02 bis 20 Gew.-%, bezogen auf das Gewicht des Trägers, beträgt.

5. Verfahren nach Anspruch 3, dadurch gekennzeichnet, dass der Dehydrierkatalysator zusätzlich 0,1 bis 20 Gew.-% Chrom, bezogen auf das Gewicht des Trägers enthält.

6. Verfahren nach Anspruch 1, dadurch gekennzeichnet, dass die Dehydrierung in einem Kohlenwasserstoff oder einem aliphatischen oder aromatischen Äther, Ester oder Amid als Lösungsmittel ausgeführt wird.

**Revendications**

1. Procédé de préparation de pyridazones-3 répondant à la formule générale:

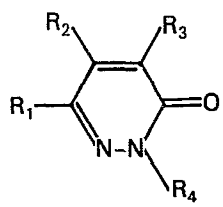

dans laquelle les radicaux $R_1$ à $R_4$ peuvent être identiques ou différents et peuvent représenter chacun l'hydrogène, un radical alkyle linéaire, ramifié ou cyclique ou un radical aryle, procédé caractérisé en ce qu'on chauffe à une température de 150 à 350 °C des dihydro-4,5 pyridazones-3 répondant à la formule générale:

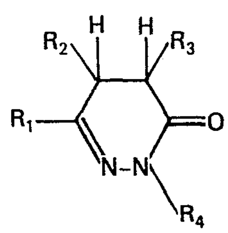

dans laquelle $R_1$ à $R_4$ ont les significations précédemment données, en phase liquide, en présence d'un catalyseur de déshydrogénation qui contient du palladium, du platine, du ruthénium, du rhodium ou de l'iridium.

2. Procédé selon la revendication 1, caractérisé en ce qu'on effectue la réaction sous une pression de 0,001 à MPa.

3. Procédé selon la revendication 1 ou 2, caractérisé en ce que le métal noble est déposé sur un support.

4. Procédé selon la revendication 3, caractérisé en ce que la concentration du métal noble est de 0,02 à 20% en poids par rapport au poids du support.

5. Procédé selon la revendication 3 ou 4, caractérisé en ce que le catalyseur de déshydrogénation contient en outre de 0,1 à 20% en poids de chrome par rapport au poids du support.

6. Procédé selon l'une quelconque des revendications 1 à 5, caractérisé en ce qu'on effectue la déshydrogénation dans un solvant qui est un hydrocarbure ou un éther, un ester ou un amide aliphatique ou aromatique.

**Claims**

1. Process for the preparation of 3-pyridazones of the formula

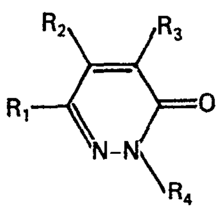

wherein the radicals $R_1$ to $R_4$, which may be the same or different, each are hydrogen, straight chain, branched or cyclic alkyl or aryl, characterized by heating 4,5-dihydro-3-pyridazones of the formula

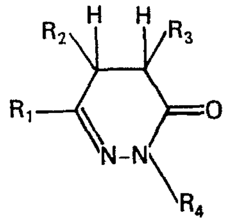

wherein $R_1$ to $R_4$ are defined as above, in the liquid phase, in the presence of a dehydrogenation catalyst containing palladium, platinum, ruthenium, rhodium or iridium, to a temperature of from 150 to 350 °C.

2. Process as claimed in claim 1, which comprises performing the reaction under a pressure of from 0.01 to 20 bars.

3. Process as claimed in claim 1, wherein the noble metal is supported on a carrier.

4. Process as claimed in claim 3, wherein the concentration of the noble metal is in the range of from 0.02 to 20 weight %, calculated on the weight of the carrier.

5. Process as claimed in claim 3, wherein the dehydrogenation catalyst contains additionally of from 0.1 to 20 weight % of chromium, calculated on the weight of the carrier.

6. Process as claimed in claim 1, which comprises carrying out the dehydrogenation in a hydrocarbon or in an aliphatic or aromatic ether, ester or amide as solvent.